(19) Europäisches Patentamt
European Patent Office
Office européen des brevets

(11) Publication number: **0 298 590 B1**

# (12) EUROPEAN PATENT SPECIFICATION

(45) Date of publication of patent specification: **11.12.91 Bulletin 91/50**

(51) Int. Cl.$^5$: **C07D 213/64,** A01N 53/00

(21) Application number: **88304255.8**

(22) Date of filing: **11.05.88**

## (54) Halogenated esters.

(30) Priority: **10.06.87 GB 8713570**

(43) Date of publication of application: **11.01.89 Bulletin 89/02**

(45) Publication of the grant of the patent: **11.12.91 Bulletin 91/50**

(84) Designated Contracting States: **AT BE CH DE ES FR GB GR IT LI LU NL SE**

(56) References cited:
EP-A- 0 112 293
EP-A- 0 145 179

(73) Proprietor: **IMPERIAL CHEMICAL INDUSTRIES PLC**
**Imperial Chemical House, Millbank**
**London SW1P 3JF (GB)**

(72) Inventor: **Salmon, Roger**
**38 Tawfield**
**Bracknell Berkshire (GB)**
Inventor: **McDonald, Edward**
**5 Grayling Close**
**Marlow Buckinghamshire (GB)**
Inventor: **Bushell, Michael John**
**21 Suffolk Close**
**Woosehill Wokingham Berkshire (GB)**

(74) Representative: **Bishop, Nigel Douglas et al**
**Imperial Chemical Industries PLC Legal Department: Patents PO Box 6 Bessemer Road**
**Welwyn Garden City Herts, AL7 1HD (GB)**

**EP 0 298 590 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to novel halogenated esters, useful as insecticides and acaricides, to compositions comprising them and to methods of combating pests therewith.

European Patent Applications Nos 112,293, 145,661 and 145,179 disclose insecticidal activity for 1-(6-phenoxypyrid-2-yl)ethyl 3-(2,2-dichloroethenyl)-2,2-dimethylcyclopropanecarboxylate and isomers thereof, and for 1-(6-phenoxypyrid-2-yl)methyl esters of 3-(3,3,3-trifluoro-2-haloprop-1-en-1-yl)-2,2-dimethylcyclopropanecarboxylic acids. We have now found that certain 1-(6-phenoxypyrid-2-yl)ethyl esters of 3-(3,3,3-trifluoro-2-haloprop-1-en-1-yl)-2,2-dimethylcyclopropanecarboxylic acids and isomers thereof exhibit high levels of insecticidal activity, and in particular show a surprisingly high level of acaricidal activity.

Accordingly, in a first aspect, the invention provides compounds of formula (I) :

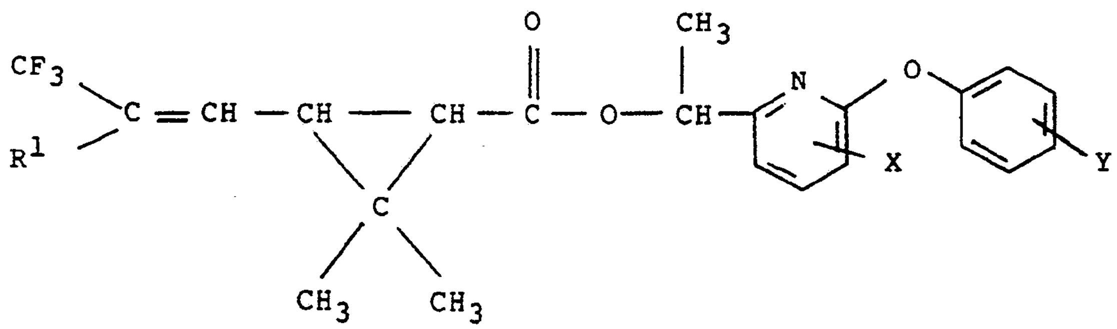

(I)

and stereoisomers thereof, wherein $R^1$ is selected from chlorine, bromine and fluorine, X is selected from hydrogen and halogen, and Y is selected from hydrogen and halogen.

The compounds of formula (I) may exist in a number of stereoisomeric forms. The carbon atoms of the 1 and 3 position in the cyclopropane ring and the alpha carbon atom in the alcohol moiety are asymmetrically substituted and may each exist in either of two configurations, the R-form or the S-form. There are, therefore, a total of eight stereoisomeric forms resulting from asymmetric substitution. In addition, the compounds of formula (I) exhibit geometric isomerism at the carbon-carbon double bond of the propenyl substituent on the cyclopropane ring, thus providing that each isomer may exist in the E-form or the Z-form. Certain isomers and mixtures of isomers of the compounds of formula (I) may exhibit higher insecticidal and acaricidal activity than others. It is to be understood that the scope of the invention includes not only mixtures of isomers of invention compounds, including racemic mixtures and enantiomer pairs, but also any individual isomer.

Examples of compounds according to the invention include those listed in Table I and stereoisomers or mixtures of stereoisomers thereof. The compounds in Table I correspond to formula (I), with specific values of $R^1$, X and Y being driven in the Table.

TABLE I

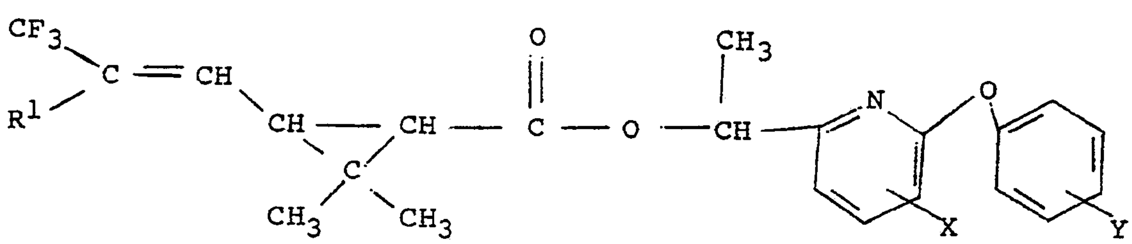

| Compound No. | $R^1$ | X | Y |
|---|---|---|---|
| 1 | Cl | H | H |
| 2 | Cl | H | Cl |
| 3 | F | H | H |
| 4 | Br | H | H |

Specific examples of compounds according to the invention include those given below :
(RS)-1-(6-phenoxypyrid-2-yl)ethyl (1RS,cis)-3-(Z-2-chloro-3,3,3-trifluoroprop-1-en-1-yl)-2,2-dimethylcyclopropanecarboxylate (Product I) ;
The more polar pair of enantiomers derived by high pressure liquid chromatographic separation of Product I, believed to be the pair of enantiomers comprising (R)-1-(6-phenoxypyrid-2-yl)ethyl (1R,cis)-3-(Z-2-chloro-3,3,3-trifluoroprop-1-en-1-yl)-2,2-dimethylcyclopropanecarboxylate and (S)-1-(6-phenoxypyrid-2-yl)ethyl (1S,cis)-3-(Z-2-chloro-3,3,3-trifluoroprop-1-en-1-yl)-2,2-dimethylcyclopropanecarboxylate (Product III) ;
(RS)-1-(6-phenoxypyrid-2-yl)ethyl (1R,cis)-3-(Z-2-chloro-3,3,3-trifluoroprop-1-en-1-yl)-2,2-dimethylcyclopropanecarboxylate (Product IV) ;
(RS)-1-(6-phenoxypyrid-2-yl)ethyl (1R,trans)-3-(Z-2-chloro-3,3,3-trifluoroprop-1-en-1-yl)-2,2-dimethylcyclopropanecarboxylate (Product V) ;
(RS)-1-(6-phenoxypyrid-2-yl)ethyl (1RS,trans)-3-(Z-2-chloro-3,3,3-trifluoroprop-1-en-1-yl)-2,2-dimethylcyclopropanecarboxylate (Product VI) ;
(RS)-1-(6-phenoxypyrid-2-yl)ethyl (1RS,trans)-3-(Z-2-chloro-3,3,3-trifluoroprop-1-en-1-yl)-2,2-dimethylcyclopropanecarboxylate (racemic enantiomer pair) (Product VII) ;
(RS)-1-[6-(4-chlorophenoxy)pyrid-2-yl]ethyl (1RS,cis)-3-(Z-2-chloro-3,3,3-trifluoroprop-1-en-1-yl)-2,2-dimethylcyclopropanecarboxylate (Product VIII) ;
(RS)-1-(6-phenoxypyrid-2-yl)ethyl (1RS,cis)-3-(Z-2,3,3,3-tetrafluoroprop-1-en-1-yl)-2,2-dimethylcyclopropanecarboxylate (Product IX) ;
(RS)-1-(6-phenoxypyrid-2-yl)ethyl (1RS,trans)-3-(Z-2,3,3,3-tetrafluoroprop-1-en-1-yl)-2,2-dimethylcyclopropanecarboxylate (Product X) ;
(RS)-1-(6-phenoxypyrid-2-yl)ethyl (1Rs,cis)-3-(Z-2-bromo-3,3,3-trifluoroprop-1-en-1-yl)-2,2-dimethylcyclopropanecarboxylate (Product XI) ;
(RS)-1-(6-phenoxypyrid-2-yl)ethyl (1RS,trans)-3-(Z-2-bromo-3,3,3-trifluoroprop-1-en-1-yl)-2,2-dimethylcyclopropanecarboxylate (Product XII).

The compounds of formula (I) are esters and may be prepared by conventional esterification processes, of which the following are examples.

(a) An acid of formula (II) :

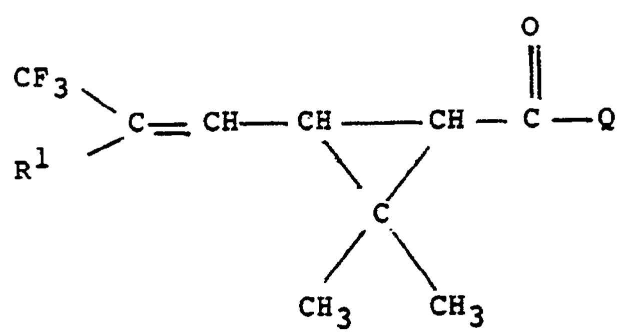

(II)

where Q represents the hydroxy group and $R^1$ is selected from chlorine, bromine and fluorine, may be reacted directly with an alcohol of formula (III) :

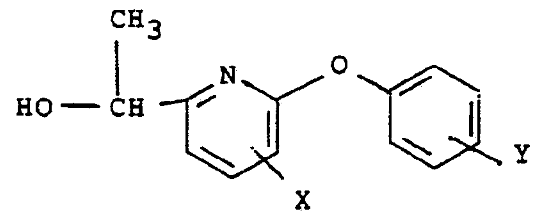

(III)

where X and Y have any of the meanings given hereinbefore, the reaction preferably taking place in the presence of an acid catalyst, for example, dry hydrogen chloride, or a dehydrating agent, for example, a carbodiimide such as dicyclohexylcarbodiimide.

(b) An acid halide of formula (II) where Q represents a halogen atom, preferably a chlorine atom, may be reacted with an alcohol of formula (III), the reaction preferably taking place in the presence of a base, for example, pyridine, trialkylamine, alkali metal hydroxide or carbonate, or alkali metal alkoxide.

(c) An acid of formula (II) where Q represents the hydroxy group or, preferably, an alkali metal salt thereof, may be reacted with a compound of formula (IV) :

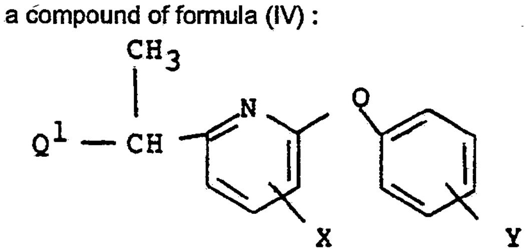

(IV)

where $Q^1$ represents a halogen atom, preferably bromine or chlorine, or $Q^1$ represents the mesylate or tosylate group, and X and Y have any of the meanings given hereinbefore, or with a quaternary ammonium salt derived from the reaction between such halides and a tertiary amine, for example pyridine, or a trialkylamine such as triethylamine.

(d) A lower alkyl ester of formula (II) where Q represents a lower alkoxy group containing up to six carbon atoms, preferably the methoxy or ethoxy group, is heated with the alcohol of formula (III) where X and Y have any of the meanings given hereinbefore, to effect a transesterification reaction. Preferably the process is performed in the presence of a suitable catalyst, for example, an alkali metal alkoxide, such as sodium methoxide, or an alkylated titanium derivative, such as tetramethyl titanate.

All of these conventional processes for the preparation of esters may be carried out using solvents and diluents for the various reactants where appropriate, and may be accelerated or lead to higher yields of product when performed at elevated temperatures or in the presence of appropriate catalysts, for example phase-transfer catalysts.

The preparation of individual isomers or mixtures of isomers of the compounds of formula (I) may be carried out in the same manner but commencing from the corresponding individual isomers of compounds of formula (II) and formula (III). These may be obtained by conventional isomer separation techniques from mixtures of isomers. Thus, for example, cis and trans isomers of carboxylic acids of formula (II), wherein Q represents the hydroxy group, may be separated by fractional crystallisation of the acids or salts thereof, whilst the various optically active species may be obtained by fractional crystallisation of salts of the acids with optically active amines, followed by regeneration of the optically active acid, which may then be esterified with alcohols of formula (III) by the processes described hereinabove. The alcohols of formula (III) may also be resolved into optically active species by standard procedures prior to esterification, thus providing access to any isomer or mixture of isomers of the compounds of formula (I). Mixtures of isomers of the esters of formula (I) may themselves be subjected to separation techniques to provide specific isomers or mixtures of isomers. Diastereoisomeric mixtures may be separated by physical means, for example by chromatography or recrystallisation. Such separation techniques may produce single enantiomers, one or more pairs of enantiomers, or mixtures of diastereoisomers. Where pairs of enantiomers are produced, they may be in the form of racemic mixtures or they may contain an enantiomeric excess of one isomer. Further details of the preparation of esters of formula (I)

and the separation of isomers thereof are provided in the Examples driven hereinafter.

The alcohols of formula (III) may be prepared from the aldehydes of formula (V) by reaction with a Grignard reagent of formula $CH_3MgBr$, as illustrated in Scheme I :

Scheme I

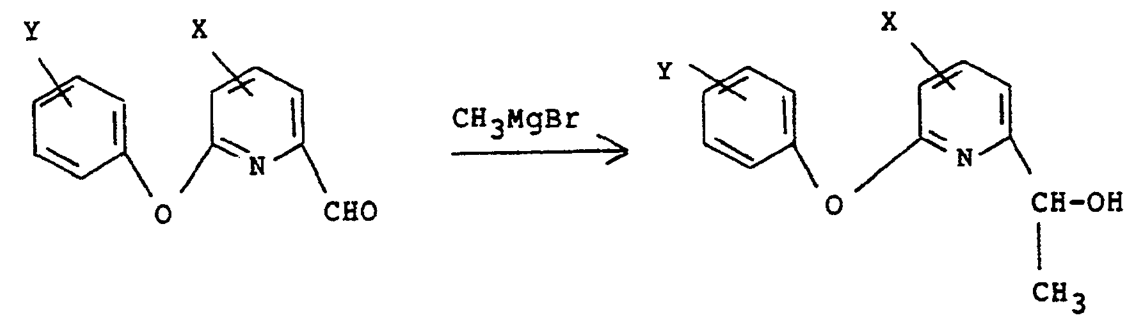


(V) (III)

The aldehydes of formula (V) may themselves be prepared by from the appropriately substituted 2-halo-6-phenoxypyridines by the method described in UK Patent Application No. 2091256. The preparation of 2-halo-6-phenoxypyridines is described in French Patent No. 1527714. The preparation of the aldehydes of formula V is also described in US Patent No. 4256893.

The aldehydes of formula (V) may also be prepared by oxidation of the corresponding alcohols of formula (VI) :

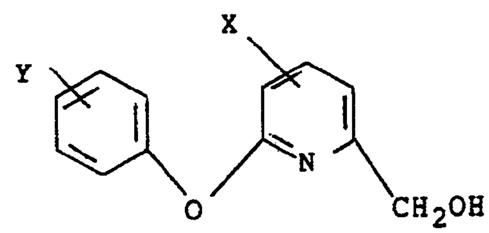

(VI)

using conventional oxidation procedures, such as the Swern oxidation described in Tetrahedron, 34, 1651-1660, (1978), or alternatively by oxidation in a mixture of an aqueous solution of an alkali metal hypohalite (for example sodium hypochlorite or sodium hypobromite) and a lower alkyl ester of a lower alkanoic acid (for example ethyl acetate), in the presence of a phase transfer catalyst (for example tetra-n-butylammonium hydrogen sulphate), in an adaptation of the method described in Tetrahedron Letters (1976) 1641 (G A Lee & H H Freedman).

This latter method is illustrated in Scheme II for the particular case of the preparation of 6-(4-chlorophenoxy)-2-formylpyridine.

Scheme II

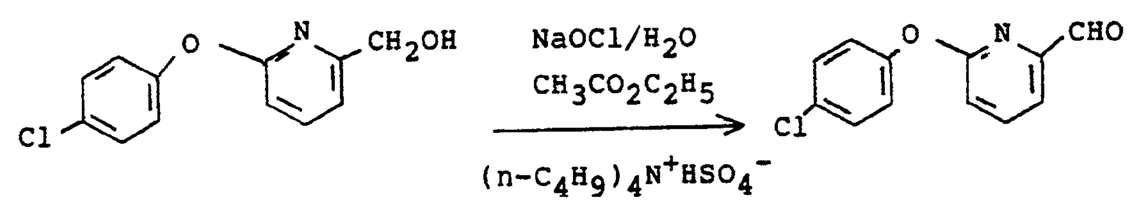

The alcohols of formula VI may be prepared according to the method described in US Patent No. 4,256,893.

The preparation of the compounds of formula II wherein Q represents hydroxy, alkoxy or halo, is fully described in UK Patent Specification No. 2,000,764 and in US Patent No. 4,183,948.

The compounds of formula (I) may be used to combat and control infestations of insect and acarine pests. The insect and acarine pests which may be combated and controlled by the use of the invention compounds include those pests associated with agriculture (which term includes the growing of crops for food and fibre products, horticulture and animal husbandry), forestry, the storage of products of vegetable origin, such as fruit, grain and timber, and also those pests associated with the transmission of diseases of man and animals.

In order to apply the compounds to the locus of the pests they are usually formulated into compositions which include in addition to the insecticidally active ingredient or ingredients of formula (I) suitable inert diluent or carrier materials, and/or surface active agents.

The compounds of the invention may be the sole active ingredient of the composition or they may be admixed with one or more additional active ingredients such as insecticides, insecticide synergist, herbicides, fungicides or plant growth regulators where appropriate.

Suitable additional active ingredients for inclusion in admixture with the compounds of the invention may be compounds which will broaden the spectrum of activity of the compounds of the invention or increase their persistence in the location of the pest. They may synergist the activity of the compounds of the invention or complement the activity for example by increasing the speed of effect, improving knockdown or overcoming repellency. Additionally multi-component mixtures of this type may help to overcome or prevent the development of resistance to individual components.

The particular insecticide, herbicide or fungicide included in the mixture will depend upon its intended utility and the type of complementary action required. Examples of suitable insecticides include the following :

(a) Pyrethroids such as permethrin, esfenvalerate, deltamethrin, cyhalothrin, biphenthrin, fenpropathrin, cyfluthrin, tefluthrin, fish safe pyrethroids for example ethofenprox, natural pyrethrins, tetramethrin, s-bioallethrin, fenfluthrin, prallethrin and 5-benzyl-3-furylmethyl-(E)-(1R,3S)-2,2-dimethyl-3-(2-oxothiolan-3-ylidenemethyl)cyclopropane carboxylate ;

(b) Organophosphates such as profenofos, sulprofos, dichlorvos, methyl parathion, azinphos-methyl, demeton-s-methyl, heptenophos, thiometon, fenamiphos, monocrotophos, profenophos, triazophos, methamidophos, dimethoate, phosphamidon, malathion, chlorpyrifos, phosalone, fensulfothion, fonofos, phorate, phoxim, pyrimiphos-methyl, fenitrothion and diazinon ;

(c) Carbamates (including aryl carbamates) such as pirimicarb, cloethocarb, carbofuran, ethiofencarb, aldicarb, thiofurox, carbosulfan, bendiocarb, fenobucarb, propoxur and oxamyl ;

(d) Benzoyl ureas such as triflumuron, chlorofluazuron ;

(e) Organic tin compounds such as cyhexatin, fenbutatin oxide, azocyclotin ;

(f) Macrolides such as avermectins or milbemycins, for example such as abamectin, avermectin, and milbemycin ;

(g) Hormones and synthetic mimics thereof such as juvenile hormone, juvabione, ecdysones, methoprene and hydroprene.

(h) Pheromones.

(i) Organochlorine compounds such as benzene hexachloride, DDT, chlordane or dieldrin.

In addition to the major chemical classes of insecticide listed above, other insecticides having particular targets may be employed in the mixture if appropriate for the intended utility of the mixture. For instance selective insecticides for particular crops, for example stemborer specific insecticides for use in rice such as cartap or buprofezin, can be employed. Alternatively insecticides or acaricides specific for particular insect species/stages for example ovolarvicides such as clofentezine, amitraz, chlordimeform, flubenzimine, hexythiazox and tetradifon, motilicides such as dicofol or propargite, adulticides such as bromopropylate, chlorobenzilate, or insect growth regulators such as hydramethylon, cyromazine, methoprene, chlorofluazuron and diflubenzuron may also be included in the compositions.

Examples of suitable insecticide synergists for use in the compositions include piperonyl butoxide, sesamex, and dodecyl imidazole.

Suitable herbicides, fungicides and plant growth regulators for inclusion in the compositions will depend upon the intended target and the effect required. An example of a rice selective herbicide which can be included is propanil, an example of a plant growth regulator for use in cotton is "Pix", and examples of fungicides for use in rice include blasticides such as blasticidin-S. The choice of other ingredients to be used in mixture with the active ingredient will often be within the normal skill of the formulator, and will be made from known alternatives depending upon the total effect to be achieved.

The ratio of the compound of the invention to any other active ingredient in the composition will depend upon a number of factors including the type of insect pests to be controlled, and the effects required from the mixture. However in general, the additional active ingredient of the composition will be applied at about the rate it would usually be employed if used on its own, or at a lower rate if synergism occurs.

The compositions may be in the form of dusting powders wherein the active ingredient is mixed with a solid diluent or carrier, for example kaolin, bentonite, kieselguhr, or talc, or they may be in the form of granules, wherein the active ingredient is absorbed in a porous granular material, for example pumice.

Alternatively the compositions may be in the form of liquid preparations to be used as dips, sprays or aerosols. Dips and sprays are generally aqueous dispersions or emulsions of the active ingredient in the presence of one or more known wetting agents, dispersing agents or emulsifying agents (surface active agents). Aerosol compositions may contain the active ingredient or ingredients, a propellant and an inert diluent, for example odourless kerosene or alkylated benzenes. In a preferred form, aerosol compositions may contain from 0.005% to 4% of active ingredient or ingredients, the remainder of the composition comprising a solvent, selected from odourless kerosine and alkylated benzenes, and a propellant. Aerosol compositions may optionally incorporate other additives, for example perfumes or corrosion inhibitors.

Wetting agents, dispersing agents and emulsifying agents may be of the cationic, anionic or non-ionic type. Suitable agents of the cationic type include, for example, quaternary ammonium compounds, for example cetyltrimethyl ammonium bromide. Suitable agents of the anionic type include, for example, soaps, salts of aliphatic monoesters or sulphuric acid, for example sodium lauryl sulphate, salts of sulphonated aromatic compounds, for example sodium dodecylbenzenesulphonate, sodium, calcium or ammonium lignosulphonate, or butylnaphthalene sulphonate, and a mixture of the sodium salts of diisopropyl- and triisopropylnaphthalene sulphonates. Suitable agents of the non-ionic type include, for example, the condensation products of ethylene oxide with fatty alcohols such as oleyl alcohol or cetyl alcohol, or with alkyl phenols such as octyl phenol, nonyl phenol and octyl cresol. Other non-ionic agents are the partial esters derived from long chain fatty acids and hexitol anhydrides, the condensation products of the said partial esters with ethylene oxide, and the lecithins.

The compositions may be prepared by dissolving the active ingredient in a suitable solvent, for example, a ketonic solvent such as diacetone alcohol, or an aromatic solvent such as trimethylbenzene and optionally adding the mixture so obtained to water which may contain one or more known wetting, dispersing or emulsifying agents.

Other suitable organic solvents are dimethyl formamide, ethylene dichloride, isopropyl alcohol, propylene glycol and other glycols, diacetone alcohol, toluene, kerosene, white oil, methylnaphthalene, xylenes and trichloroethylene, N-methyl-2-pyrrolidone and tetrahydrofurfuryl alcohol (THFA).

The compositions which are to be used in the form of aqueous dispersions or emulsions are generally supplied in the form of a concentrate containing a high proportion of the active ingredient or ingredients, the said concentrate to be diluted with water before use. These concentrates are often required to withstand storage for prolonged periods and after such storage, to be capable of dilution with water to form aqueous preparations which remain homogenous for a sufficient time to enable them to be applied by conventional spray equipment. The concentrates may contain 1-99% by weight of the active ingredient or ingredients. When diluted to form aqueous preparations such preparations may contain varying amounts of the active ingredient depending upon the purpose for which they are to be used. For agricultural or horticultural purposes, an aqueous preparation containing between 0.0001% and 0.1% by weight of the active ingredient is particularly useful.

In use the compositions are applied to the pests, to the locus of the pests, to the habitat of the pests, or to growing plants liable to infestation by the pests, by any of the known means of applying pesticidal compositions, for example, by dusting or spraying.

The compounds of formula (I) and compositions comprising them are very toxic to wide varieties of insect, acarine and other invertebrate pests, including, for example, the following :

<u>Myzus persicae</u> (aphids)
<u>Aphis fabae</u> (aphids)
<u>Megoura viceae</u> (aphids)
<u>Aedes aegypti</u> (mosquitos)
<u>Anopheles</u> spp. (mosquitos)
<u>Culex</u> spp. (mosquitos)
<u>Dysdercus fasciatus</u> (capsids)
<u>Musca domestica</u> (houseflies)
<u>Pieris brassicae</u> (white butterfly, larvae)
<u>Plutella maculipennis</u> (diamond back moth, larvae)
<u>Phaedon cochleariae</u> (mustard beetle)
<u>Aonidiella</u> spp. (scale insects)

Trialeuroides spp. (white flies)
Blattella germanica (cockroaches)
Periplaneta americana (cockroaches)
Blatta orientalis (cockroaches)
Spodoptera littoralis(cotton leaf worm)
Heliothis virescens (tobacco budworms)
Chortiocetes terminifera (locusts)
Diabrotica spp. (rootworms)
Agrotis spp. (cutworms)
Chilo partellus (maize stem borers)
Nilaparvata lugens (plant hoppers)
Nephotettix cincticeps (leaf hoppers)
Panonychus ulmi
Panonychus citri
Tetranychus urticae (red spider mite)
Tetranychus cinnabarinus (carmine spider mite)

The compounds according to formula (I) and compositions comprising them have been shown to be useful for the control of lepidopteran pests, for example Spodoptera spp. and Heliothis spp. and public health pests such as flies, mosquitos and cockroaches.

The compounds according to formula (I) and compositions comprising them exhibit a high level of acaricidal activity, and are particularly useful in the control of the following acarine pests :

Panonychus spp., for example Panonychus ulmi and Panonychus citri ;
Tetranychus spp., for example Tetranychus urticae and Tetranychus cinnabarinus ;
Phyllocoptruta oleivora ;
Polyphagotarsonemus spp., and
Brevipalpus spp.

They may also be useful in combating insect and acarine pests which infest domestic animals, such as Lucilia sericata, and ixodid ticks such as Boophilus spp., Ixodes spp., Amblyomma spp., Rhipicephalus spp. and Dermocentor spp. They are effective in combating both susceptible and resistant strains of these pests in their adult, larval and intermediate stages of growth, and may be applied to the infested host animal by topical, oral or parenteral administration.

The following Examples illustrate various aspects of this invention. In the preparation Examples the products were usually identified and characterised by means of nuclear magnetic reasonance (NMR) spectroscopy and infra red (IR) spectroscopy. In each case where a product is specifically named its spectral characteristics are consistent with the assigned structure. Except where stated otherwise, exemplified compounds having one or more asymmetrically substituted carbon atoms were prepared in racemic form.

In the Examples, Gas Liquid Chromatography (GLC) retention times were determined on a Hewlett Packard 5890 Gas Chromatograph, using a Chrompak, CPSil 5CB column of 12.5 M length and 0.2 mm internal diameter. Unless otherwise stated, the injection temperature was 100°C, and a temperature gradient of 15°C/minute employed, up to a maximum temperature of 280°C, maintained for 4 minutes. The carrier gas was helium at a column head pressure maintained at 11 psi. Alternative injection and maximum temperatures are indicated in the Examples where appropriate.

$^1$H Nuclear Magnetic Resonance (NMR) spectrometry was performed at a frequency of 270 MHz on a Jeol FX 270 NMR spectrometer, unless otherwise indicated. 90 MHZ, 60 MHz, and 400 MHz $^1$H NMR spectrometry were performed using Jeol FX 90Q, Jeol PMX 60SI, and Jeol GX400 spectrometers respectively.

$^{19}$F NMR spectrometry was performed on a Jeol FX90Q spectrometer at a frequency of 84.26 MHZ. All NMR shift ($\delta$) values are quoted in ppm relative to a standard (TMS or $CFCl_3$).

Molecular Ion ($M^+$) peaks were determined on one of three mass spectrometers : Jeol DX303, Kratos MS80 or Hewlett Packard HP 5992.

The following Examples illustrates various aspects of the invention.

## EXAMPLE 1

This Example illustrates the preparation of (RS)-1-(6-phenoxypyrid-2-yl)ethyl (1RS,cis)-3-(Z-2-chloro-3,3,3-trifluoroprop-1-en-1-yl)-2,2-dimethylcyclopropane-carboxylate, Product I, as a 1 : 1 mixture of enantiomer pairs.

Method A

A solution of dicyclohexylcarbodiimide (0.51 g) in dry dichloromethane (5 $cm^3$) was added to a well stirred mixture of 1-(6-phenoxypyrid-2-yl)ethanol (0.5g), (1RS, cis)-3-(Z-2-chloro-3,3,3-trifluoroprop-1-en-1-yl)-2,2-dimethylcyclopropanecarboxylic acid (0.6 g), and dichloromethane (15 $cm^3$) containing a trace amount (ca. 1-2 mg) of 4-(N,N-dimethylamino)pyridine, at the ambient temperature (ca. 20°C). After 1 hour the precipitate was removed by filtration and the filtrate concentrated by evaporation of the solvent under reduced pressure. The residual oil was subjected to column chromatography using a silica gel column eluted with a 1 : 5 mixture (by volume) of diethyl ether and n-hexane to yield (RS)-1-(6-phenoxypyrid-2-yl)ethyl (1RS, cis)-3-(Z-2-chloro-3,3,3-trifluoroprop-1-en-1-yl)-2,2-dimethylcyclopropanecarboxylate (0.7 g). The NMR spectrum is consistent with the product being in the form of a 1 : 1 mixture of the two racemic pairs of enantiomeric isomers.

| | |
|---|---|
| $^1H$ NMR ($CDCl_3$) : | 1.20 (s, 3H) ; 1.32 (bs, 9H) ; 1.57 (bs, 6H) ; 2.0-2.3 (m, 4H) ; 5.77 (q, 2H) ; 6.6-7.8 (m, 18H). |
| Infra red (liquid film) : | 3090, 2980, 2950, 1733, 1600, 1582, 1450, 1300, 1280, 1210, 1150 $cm^{-1}$. |

Method B

(1RS, cis)-1-Chlorocarbonyl-3-(Z-2-chloro-3,3,3-trifluoroprop-1-en-1-yl)-2,2-dimethylcyclopropane (14.8 g) was added to a stirred mixture of 1-(6-phenoxypyrid-2-yl)ethanol (16.7 g), pyridine (6.86 g) and toluene (190 $cm^3$) over a period of 7 minutes whilst the temperature of the mixture was maintained in the range 21 to 26°C by external cooling. The resultant mixture was stirred at the ambient temperature for 2.5 hours and allowed to stand for 16 hours. Water (400 $cm^3$) was added to the mixture with vigorous stirring after which the phases were allowed to separate. The toluene phase was collected and the aqueous phase extracted with toluene (200 $cm^3$). The combined toluene extracts and the toluene phase were washed with water (3 × 200 $cm^3$) and with saturated aqueous sodium bicarbonate solution, and dried over anhydrous magnesium sulphate. The solvent was removed by evaporation under reduced pressure and the residual oil subjected to column chromatography using a silica gel column (Woelm TSC) eluted with a 1 : 9 mixture by volume of ethyl acetate and n-hexane. The product (25 g) was obtained as a pale yellow oil, identical by n.m.r. and infra red spectroscopy with the product of Method A above.

EXAMPLE 2

This Example illustrates the separation of the product of Example 1 into its two constituent racemic pairs of enantiomeric isomers.

(RS)-1-(6-Phenoxypyrid-2-yl)ethyl (1RS, cis)-3-(Z-2-chloro-3,3,3-trifluoroprop-1-en-1-yl)-2,2-dimethylcyclopropanecarboxylate (22 × 14 mg aliquots) were separated by the high pressure liquid chromatography technique using a ZORBAX-SIL column (DuPont), having a diameter of 21.2 mm and length of 25 cm eluted with a 49 : 1 mixture (by volume) of n-hexane and tetra-hydrofuran, and using a U.V. detector set at 254 nm. Two fractions were obtained. The less polar fraction (Product II, 160 mg, 100% by g.l.c.) is believed to be the racemic pair of enantiomeric isomers consisting of (R)-1-(6-phenoxypyrid-2-yl)ethyl (1S, cis)-3-(Z-2-chloro-3,3,3-trifluoroprop-1-en-1-yl)-2,2-dimethylcyclopropane-carboxylate and (S)-1-(6-phenoxypyrid-2-yl)ethyl (1R, cis)-3-(Z-2-chloro-3,3,3-trifluoroprop-1-en-1-yl)-2,2-dimethylcyclopropanecarboxylate. The more polar fraction (Product III, 120 mg, obtained as a mixture with ca. 78 by weight of the less polar fraction) is believed to be the racemic pair of enantiomeric isomers consisting of (S)-1-(6-phenoxypyrid-2-yl)ethyl (1S, cis)-3-(Z-2-chloro-3,3,3-trifluoroprop-1-en-1-yl)-2,2-dimethylcyclopropane-carboxylate and (R)-1-(6-phenoxypyrid-2-yl)ethyl (1R, cis)-3-(Z-2-chloro-3,3,3-trifluoroprop-1-en-1-yl)-2,2-dimethylcyclopropanecarboxylate.

Less Polar Fraction —

| | |
|---|---|
| $^1H$ NMR ($CDCl_3$) (270 MHz) : | 1.30 (d, 6H) ; 1.55 (d, 3H) ; 2.05 (d, 1H) ; 2.15 (t, 1H) ; 5.77 (q, 1H) ; 6.7 (d, 1H); 6.85 (d, 1H) ; 6.95 (d, 1H) ; 7.1-7.2 (m, 3H) ; 7.38 (t, 2H) ; 7.65 (t, 1H). |

More Polar Fraction — Product III

| | |
|---|---|
| $^1H$ NMR ($CDCl_3$) : | 1.20(s) ; 1.30(s) ; 1.55(m) ; 2.05-2.2(m) ; 5.77(q) ; 6.7(d) ; 6.85(d) ; 6.90(d) ; 7.0(d) ; 7.1-7.2(m) ; 7.3-7.4(m) ; 7.6-7.7(m). |

The integration of this spectrum is consistent with there being a small quantity ( < 10%) of the less polar

isomer present.

When the two fractions were evaluated for contact acaricidal activity against the spider mite Tetranychus urticae it was found that the more polar fraction showed 25-30 times the activity of the less polar fraction.

EXAMPLE 3

The following compounds were prepared from (RS)-1-(6-phenoxypyrid-2-yl)ethanol and the appropriate isomer of 2-chloro-3,3,3-trifluoroprop-1-en-1-yl)-2,2-dimethylcyclopropanecarboxylic acid using the procedure described in Example 1, Method A.

(i) (RS)-1-(6-phenoxypyrid-2-yl)ethyl (1R, cis)-3-(Z-2-chloro-3,3,3-trifluoroprop-1-en-1-yl)-2,2-dimethylcyclopropanecarboxylate, Product IV, as a 1 : 1 mixture of diastereoisomers.
(NOTE : purification of the crude reaction product was achieved by column chromatography on a silica gel support, eluting with a 10 : 1 mixture (by volume) of n-hexane and ethyl acetate).

| | |
|---|---|
| $^1$H NMR ($CDCl_3$) (ppm) : | 1.3 (6H, broad s) ; 1.55 (3H, d) ; 2.05 (1H, d) ; 2.15 (1H, t) ; 5.8 (1H, q) ; 6.7 (1H, d) ; 6.9 (1H, q) ; 7.0 (1H, d) ; 7.15 (3H, m) ; 7.4 (2H, m) ; 7.65 (1H, m). |
| Infra red (liquid film) : | 3090, 2980, 2950, 1730, 1600, 1580, 1445, 1300, 1275, 1205, 1140 $cm^{-1}$. |
| Molecular Ion : | 439. |
| GLC Retention Time : | 9.19 minutes<br>9.36 minutes<br>(mixture of two diastereoisomers). |

(ii) (RS)-1-(6-phenoxypyrid-2-yl)ethyl (1R-trans)-3-(Z-2-chloro-3,3,3-trifluoroprop-1-en-1-yl)-2,2-dimethylcyclopropanecarboxylate, Product V, as a 1 : 1 mixture of diastereoisomers.
(NOTE : purification of the crude reaction product was achieved by column chromatography on a silica gel support, eluting with a 10 : 1 mixture (by volume) of n-hexane and ethyl acetate).

| | |
|---|---|
| 400 MHz $^1$H NMR ($CDCl_3$) (ppm) : | 1.3 (6H, broad s); 1.55 (3H, d); 1.85 (1H, d); 2.4 (1H, m); 5.8 (1H, q); 6.15 (1H, m); 6.7 (1H, d); 7.05 (1H, m); 7.1-7.2 (3H, m); 7.4 (2H, m); 7.65 (1H, m). |
| Infra red (liquid film) : | 3090, 2980, 2930, 1730, 1600, 1595, 1445, 1300, 1280, 1220, 1170 $cm^{-1}$. |
| GLC Retention Time : | 7.8 minutes<br>7.91 minutes<br>(mixture of two diastereoisomers). |

(iii) (RS)-1-(6-phenoxypyrid-2-yl)ethyl (1RS-trans)-3-(Z-2-chloro-3,3,3-trifluoroprop-1-en-1-yl)-2,2-dimethylcyclopropanecarboxylate, as a 4 : 3 mixture of the two racemic pairs of enantiomers.
(NOTE : in this case, diethyl ether was added to the reaction mixture, which was then filtered. Evaporation of the solvents under reduced pressure have the title compound as a white solid, shown by GLC analysis to be 77% pure, in the form of a 4 : 3 mixture of enantiomer pairs).

| | |
|---|---|
| GLC Retention Time : | 8.59 minutes<br>8.69 minutes<br>(4 : 3 mixture of enantiomer pairs). |

EXAMPLE 4

This Example illustrates the separation of isomers of (RS)-1-(6-phenoxypyrid-2-yl)ethyl (1RS-trans)-3-(Z-2-chloro-3,3,3-trifluoroprop-1-en-1-yl)-2,2-dimethylcyclo-propanecarboxylate.

The product of Example 3 (iii) was dissolved in a small quantity of dichloromethane and applied to a short

silica gel column. The column was eluted with dichloromethane, and separate fractions collected. The major product containing fractions contained the mixture of diastereoisomers of Example 3 (iii) in approximately 85% purity (by GLC analysis). Evaporation of the eluent under reduced pressure gave a gum which was triturated in a small volume of n-hexane and cooled to 0°C. On standing at this temperature, a crystalline precipitate was obtained. The precipitate was isolated and shown by GLC analysis to be the enantiomer pair (Product VI — configuration not assigned) having the shorter retention time, in 89% purity. Evaporation of the solvent from the mother liquors have a colourless gum, shown by GLC analysis to be an approximately 5 : 4 mixture of the two enantiomer pairs (Product VII) in 94% purity.

Product VI — Racemicenantiomer pair

| | |
|---|---|
| $^1$H NMR ($CDCl_3$) (ppm) : | 1.25 (3H, s) ; 1.35 (3H, s) ; 1.55 (3H, d) ; 1.85 (1H, d) ; 2.4 (1H, m) ; 5.8 (1H, q) ; 6.15 (1H, d) ; 6.7 (1H, d) ; 7.05 (1H, d) ; 7.15 (3H, m) ; 7.4 (2H, m) ; 7.65 (1H, t). |
| Melting Point : | 70°C. |

Characterised as a single, racemic enantiomer pair of (RS)-1-(6-phenoxypyrid-2-yl)ethyl (1RS-trans)-3-(Z-2-chloro-3,3,3-trifluoroprop-1-en-1-yl)-2,2-dimethylcyclopropanecarboxylate.

Product VII — 5 : 4 mixture of enantiomer pairs

| | |
|---|---|
| $^1$H NMR ($CDCl_3$) (ppm) : | 1.25 (6H, broad s) ; 1.55 (3H, d) ; 1.9 (1H, d) ; 2.4 (1H, m) ; 5.8 (1H, q) ; 6.15 (1H, m) ; 6.7 (1H, d) ; 7.05 (1H, m) ; 7.15 (3H, m) ; 7.4 (2H, m) ; 7.65 (1H, m). |
| Infra red (liquid film) : | 3090, 2980, 2920, 1730, 1600, 1580, 1445, 1300, 1280, 1220, 1170 $cm^{-1}$. |
| Molecular Ion : | 439. |

Characterised as a 5 : 4 mixture of the two, racemic enantiomer pairs of (RS)-1-(6-phenoxypyrid-2-yl)ethyl (1RS-trans)-3-(Z-2-chloro-3,3,3-trifluoroprop-1-en-1-yl)-2,2-dimethylcyclopropanecarboxylate.

EXAMPLE 5

This Example illustrates the stages in the preparation of (RS)-1-[6-(4-chlorophenoxy)pyrid-2-yl]ethyl (1RS,cis)-3-(Z-2-chloro-3,3,3-trifluoroprop-1-en-1-yl)-2,2-dimethylcyclopropanecarboxylate, Product VIII, as a 3 : 2 mixture of enantiomer pairs.

Stage 1 : preparation of 6-(4-chlorophenoxy)pyridine-2-carboxaldehyde

A solution of 6-(4-chlorophenoxy)pyridine-2-methanol (0.43 g) in ethyl acetate (5 $cm^3$) containing, as phase transfer catalyst, tetra-n-butylammonium hydrogen sulphate (0.005 g) was stirred at the ambient temperature and treated with a solution of sodium hypochlorite (1.5 $cm^3$ of "chloros" ; 10% w/w NaOCl). The pale green mixture was stirred for 2 hours and further "chloros" (0.3 $cm^3$) added. After a further 1 hour at the ambient temperature the mixture was acidified by addition of aqueous hydrochloric acid (2 molar), and extracted with diethyl ether (100 $cm^3$). The organic layer was separated and washed with dilute aqueous sodium bicarbonate solution and water, and was then dried over anhydrous magnesium sulphate. The solvent was evaporated under reduced pressure to yield an oil. The required product was obtained by fractionating the oil using a bed of silica gel (Merck 7729) and eluting with a hexane/diethyl ether mixture (4 : 1 by volume). The product was obtained as a colourless oil (0.23 g) and was shown by infra red spectrometry and gas chromatography-mass spectrometry to be 6-(4-chlorophenoxy)pyrimidine-2-carboxaldehyde.

Stage 2 : Preparation of (RS)-1-[6-(4-chlorophenoxy)pyrid-2-yl]ethanol.

A solution of 6-(4-chlorophenoxy)pyrimidine-2-carboxaldehyde (0.23g) in dry tetrahydrofuran (5 $cm^3$) was cooled using an ice/salt bath, and stirred under a nitrogen atmosphere. To this solution was added dropwise a solution of methyl magnesium chloride (0.35 $cm^3$ of a 3 molar solution in tetrahydrofuran, available from the Aldrich Chemical Co, Gillingham, Dorset, England). The reaction mixture was stirred at 0°C, then allowed to warm to the ambient temperature over ca. 2 hours, and kept at this temperature for 18 hours. The reaction mixture was treated with saturated aqueous ammonium chloride solution and the product was extracted with diethyl

ether (2 × 100 $cm^3$). The organic layers were combined and washed with water (20 $cm^3$) and dried over anhydrous magnesium sulphate. The solvent was evaporated under reduced pressure to give an oil, which was fractionated by filtering through a bed of silica gel (Merck 7729) using (1) a hexane/diethyl ether mixture (10 :3 by volume) to remove traces of the starting aldehyde and (2) hexane/diethyl ether (1 : 1 by volume) to give the product as a colourless oil (0.16 g) after evaporation of the solvents. The identity of the product was confirmed by NMR, 1R and mass spectrometry as (RS)-1-[6-(4-chlorophenoxy)pyrid-2-yl]ethanol.

Stage 3 :

(1RS,cis)-3-(Z-2-chloro-3,3,3-trifluoroprop-1-en-1-yl)-2,2-dimethyl cyclopropane carboxylic acid (0.1 g) and (RS)-1-[6-(4-chlorophenoxy)pyrid-2-yl]ethanol (0.1 g) were reacted according to the method described in Example 1A to give an oil, which was fractionated by filtering through a bed of silica gel (Merck 7729) using hexane/diethyl ether (10 : 1 by volume) to give (RS)-1-[6-(4-chlorophenoxy)pyrid-2-yl]ethyl (1RS,cis)-3-(Z-2-chloro-3,3,3-trifluoroprop-1-en-1-yl)-2,2-dimethylcyclopropanecarboxylate, Product VIII, as a 3 : 2 mixture of enantiomer pairs

| | |
|---|---|
| $^1$H NMR ($CDCl_3$) (ppm) : | 1.20 and 1.30 (6H,s+m) ; 1.50 (3H,d) ; 2.05 (1H,m) ; 2.15 (1H,m) ; 5.75 (1H,m); 6.75-6.9 (2H,m) ; 7.0 (1H,m) ; 7.10-7.35 (4H,m) ; 7.65 (1H,m). |
| GLC Retention time : | 10.11 and 10.31 minutes |
| Molecular Ion : | 473 |

EXAMPLE 6

This Example illustrates the preparation of the (RS)-1-(6-phenoxypyrid-2-yl)ethyl esters of (1RS,cis)- and (1RS,trans)-3-(Z-2,3,3,3-tetrafluoroprop-1-en-1-yl)-2,2-dimethylcyclopropanecarboxylate (Products IX and X).

(RS)-1-(6-phenoxypyrid-2-yl)ethanol (0.540 g) and a mixture of (1RS,trans)- and (1RS,cis)-3-(Z-2,3,3,3-tetrafluoroprop-1-en-1-yl)-2,2-dimethylcyclopropanecarboxylic acids (0.565 g) (ratio 4 : 1) were reacted according to the method described in Example 1A. The crude product of the reaction was partially purified by filtering through a bed of silica gel (Merck 7729) using hexane/diethyl ether (4 : 1 by volume).

A sample (0.52 g) of the partially purified product was further fractionated by high pressure liquid chromatography (Du Pont 2.5 cm × 25 cm Si1100 ; eluent hexane/diethyl ether ; 6 : 1 by volume) to give (i) (RS)-1-(6-phenoxypyrid-2-yl)-ethyl (1RS,cis)-3-(Z-2,3,3,3-tetrafluoroprop-1-en-1-yl)-2,2-dimethylcyclopropane carboxylate (0.15 g ; Product IX, as a 6 : 5 mixture of enantiomer pairs, contaminated with 12% of the corresponding E-isomers) and (ii) the corresponding pure (1RS,trans)- ester (0.23 g ; Product X, as a 3 : 2 mixture of enantiomer pairs.

Product IX : 1H NMR ($CDCl_3$) (ppm): 1.20 and 1.30 (6H,s+m); 1.55 (3H,m); 2.0 (1H,m); 2.15 (1H,m); 5.75 (1H,m); 5.9-6.2 (1H,m); 6.70 (1H,d); 7.05 (1H,d); 7.1-7.45 (5H,m); 7.65 (1H,m).

Molecular Ion : 423

Product X : 1H NMR ($CDCl_3$) (ppm) : 1.21-1.3 (6H,m); 1.55 (3H,d); 1.8 (1H,t); 2.35 (1H,m); 5.25, 5.35 (1H,m); 5.8 (1H,q); 6.7 (1H,d); 7.0 (1H,m); 7.1-7.45 (5H,m); 7.65 (1H,m).

Molecular Ion : 423

EXAMPLE 7

This Example illustrates the preparation of the (RS)-1-(6-phenoxypyrid-2-yl)ethyl esters of (1RS,cis)- and (1RS,trans)-3-(Z-2-bromo-3,3,3-trifluoroprop-1-en-1-yl)-2,2-dimethylcyclopropanecarboxylate (Products XI and XII).

A 1 : 1 mixture of (1RS,cis)- and (1RS,trans)-3-(Z-2-bromo-3,3,3-trifluoroprop-1-en-1-yl)-2,2-dimethylcyclo-propanecarboxylic acids was esterified with (RS)-1-(6-phenoxypyrid-2-yl)ethanol according to the method described in Example 1A.

The crude reaction product was fractionated by filtering through a bed of silica gel (Merck 7729) using hexane/diethyl ether (ration 4 : 1 by volume) as eluent, to yield a colourless oil. The oil was further fractionated using HPLC (Du Pont 2.5 cm × 25 cm Si1100) using (i) hexane/diethyl ether (6 : 1 by volume) to obtain (RS)-1-(6-phenoxypyrid-2-yl)ethyl (1RS,trans)-3-(Z-2-bromo-3,3,3-trifluoroprop-1-en-1-yl)-2,2-dimethylcyclopropane-carboxylate, Product XII, as a 3 : 2 mixture of enantiomer pairs, and (ii) using hexane/tetrahydrofuran (50 : 1 by volume) to obtain (RS)-1-(6-phenoxypyrid-2-yl)ethyl (1RS,cis)-3-(Z-2-bromo-3,3,3-trifluoroprop-1-en-1-yl)-2,2-dimethylcyclopropanecarboxylate, Product XI, as a 3 : 2 mixture of enantiomer pairs.

Product XII : 1H NMR ($CDCl_3$) (ppm) : 1.25-1.35 (6H,m); 1.55 (3H,d); 1.85 (1H,m); 2.35 (1H,m); 5.85 (1H,m); 6.4 (1H,m); 6.7 (1H,d); 7.05-7.4 (6H,m); 7.65 (1H,m).

Molecular Ion : 483

Product XI : 1H NMR ($CDCl_3$) (ppm) : 1.2-1.30 (6H,m); 1.55 (3H,d); 2.05-2.15 (2H,m); 5.75 (1H,m); 6.70 (1H,d); 7.0-7.4 (7H,m); 7.65 (1H,m).

GLC Retention Time : 10.44 and 10.62 mins

Molecular Ion : 483

EXAMPLE 8

This Example illustrates the insecticidal and acaricidal activity of the compounds according to the invention.

The activity of the Product was determined using a variety of insect pests. The Product was used in the form of liquid preparations containing from 100 to 500 parts per million (ppm) by weight of the Product. The preparations were made by dissolving the Product in acetone and dilution the solutions with water containing 0.1% by weight of a wetting agent sold under the trade name "SYNPERONIC" NX until the liquid preparations contained the required concentration of the Product. "SYNPERONIC" is a Registered Trade Mark.

The test procedure adopted with regard to each pest was basically the same and comprised supporting a number of the pests on a medium which was usually a host plant or a foodstuff on which the pests feed, and treating either or both the pests and the medium with the preparations. The mortality of the pests was then assessed at periods usually varying from one to seven days after the treatment.

In the case of the species Musca domestica (housefly), an additional assessment to determine the knockdown effect of the compounds was performed. Details are given in Table II.

The results of the tests are given in Table III for each of the Products, at the rate in parts per million given in the second column, as a grading of mortality designated as A, B or C wherein A indicates 80-100% mortality or knockdown, B indicates 50-79% mortality or knockdown and C indicates less than 50% mortality or knockdown.

In Table III the pest organism used is designated by a letter code and the pests species, the support medium or food, and the type and duration of test is given in Table II.

TABLE II

| CODE LETTERS (Table III) | TEST SPECIES | SUPPORT MEDIUM/FOOD | TYPE OF TEST | DURATION (days) |
|---|---|---|---|---|
| TUa | Tetranychus urticae (spider mites - adult) | French bean leaf | Contact | 3 |
| TUe | Tetranychus urticae (spider mites - ova) | French bean leaf | Contact | 6 |
| MP | Myzus persicae (aphids) | Chinese Cabbage leaf | Contact | 3 |
| NC | Nephotettix cincticeps (green leaf hopper - nymphs) | Rice plant | Contact | 3 |
| HV | Heliothis virescens (tobacco budworm - larvae) | Cotton leaf | Residual | 3 |
| DB | Diabrotica balteata (rootworm larvae) | Filter paper/ maize seed | Residual | 3 |

TABLE II (CONT/D)

| CODE LETTERS (Table III) | TEST SPECIES | SUPPORT MEDIUM/FOOD | TYPE OF TEST | DURATION (days) |
|---|---|---|---|---|
| BG/R | Blattella germanica (cockroach nymphs) | Plastic pot | Residual | 3 |
| BG/C | Blattella germanica (cockroach nymphs) | Plastic pot | Contact | 2 |
| MD | Musca domestica (houseflies - adults) | Cotton wool/ sugar | Contact | 1 |
| MD/KD | Musca domestica (houseflies - adults) | Cotton wool/ sugar | Knockdown | 4 hours |
| SP | Spodoptera exigua (lesser army worm - larvae) | Cotton leaf | Residual | 3 |

"Contact" test indicates that both pests and medium were treated and "residual" indicates that the medium was treated before infestation with the pests.

TABLE III

| PRODUCT | RATE | MP | NC | HV | DB | BG/R | BG/C | MD | MD/KD | SP | TUa | TUe |
|---|---|---|---|---|---|---|---|---|---|---|---|---|
| I | 500 | A | – | A | A | A | – | A | – | – | A | A |
| III | 500 | A | – | A | A | A | – | A | A | – | – | – |
| IV | 500 | A | – | A | B | A | – | A | A | A | A | A |
| V | 500 | A | A | A | A | A | – | A | A | A | A | B |
| VII | 500 | A | – | A | A | A | – | A | A | A | A | A |
| VIII | 500 | A | A* | A* | A* | – | A | A** | A | A* | A | C |
| IX | 500 | A | A* | A* | A* | – | A | A** | A | A* | A | A |
| X | 500 | A | A* | A* | A* | – | A | A** | A | A* | A | A |
| XI | 500 | A | A* | A* | A* | – | A | A** | A | A* | A | A |
| XII | 500 | A | A* | A* | A* | – | A | A** | A | A* | A | A |

* indicates test duration of 2 days
** indicates test duration of 3 days

EXAMPLE 9

This Example illustrates the acaricidal properties of the compounds according to the invention.

(i) Contact activity against Tetranychus urticae.

Test chemicals were sprayed onto french bean leaves previously infested with adult Tetranychus urticae. Replicated assessments of mortality were made 3 days after treatment at a range of concentrations for each test chemical and also for a standard acaricide (bifenthrin, 3-phenyl-2-methylbenzyl (1RS,cis)-3-(Z-2-chloro-3,3,3-trifluoroprop-1-en-1-yl)-2,2-dimethylcyclopropanecarboxylate). $LC_{50}$ values (the concentration required to produce 50% mortality of the test insects) were calculated for each test chemical and also for the standard chemical. The results are expressed in Table IV as the relative potency compared with bifenthrin calculated for each test chemical according to the formula :

$$\text{Relative Potency} = \frac{LC_{50} \text{ of Test Chemical}}{LC_{50} \text{ of bifenthrin}}$$

TABLE IV

| Test Chemical | Relative Potency |
|---|---|
| I | 0.9 |
| III | 1.53 |

TABLE IV (cont)

| Test Chemical | Relative Potency |
|---|---|
| IV | 2.4 |
| V | 0.92 |
| VII | 0.4 |
| VIII | 0.66 |
| IX | 1.3 |
| X | 0.43 |
| XI | 0.84 |
| XII | 0.35 |
| Prior Art Compound A | 0.31 |
| Prior Art Compound B | 0.13 |

Prior Art compound A : (RS)-1-(6-phenoxypyrid-2-yl)ethyl (1RS,cis)-3-(2,2-dichloroethenyl)-2,2-dimethylcyclopropane carboxylate (disclosed in EP 112,293).

Prior Art compound B : (RS)-1-cyano-1-(6-phenoxypyrid)-2-yl)methyl (1RS,cis)-3-(Z-2-chloro-3,3,3-trifluoroprop-1-en-1-yl)-2,2-dimethylcyclopropanecarboxylate (disclosed in EP 145,179).

Residual Activity against Tetranychus urticae.

Test chemicals were sprayed onto french bean leaves. The leaves were allowed to dry and were then infested with adult Tetranychus urticae, and replicated assessments of mortality were made 3 days after treatment for a range of concentrations of test chemical and also for a standard acaricide (bifenthrin). The results are expressed in Table V as relative potency compared with bifenthrin, calculated as described for the contact activity test in (i) above.

TABLE V

| Test Chemical | Relative Potency |
| --- | --- |
| I | 0.7 |
| III | 1.28 |
| IV | 1.6 |
| V | 0.3 |
| VII | 0.6 |
| Prior Art Compound B | 0.07 |

Prior Art compound B : (RS)-1-cyano-1-(6-phenoxypyrid)-2-yl)methyl (1RS,cis)-3-(Z-2-chloro-3,3,3-trifluoroprop-1-en-1-yl)-2,2-dimethylcyclopropanecarboxylate (disclosed in EP 145,179).

## Claims

1. A compound of formula :

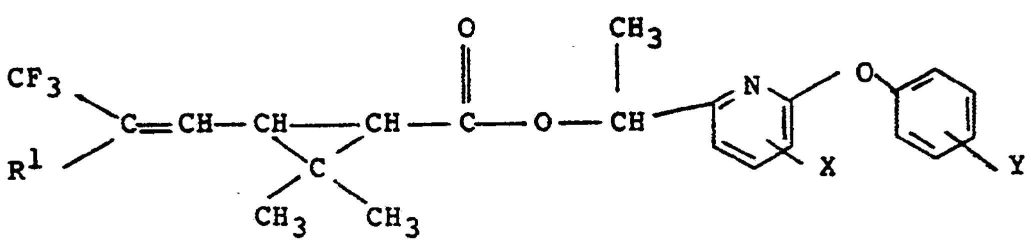

and stereoisomers thereof, wherein $R^1$ is selected from chlorine, bromine and fluorine, X is selected from hydrogen and halogen and Y is selected from hydrogen and halogen.

2. A compound according to claim 1, wherein Y is selected from hydrogen and chlorine, and stereoisomers thereof.

3. A compound according to claim 1 or claim 2 wherein $R^1$ represents chlorine or bromine, and stereoisomers thereof.

4. A compound selected from the group of compounds consisting of :

1-(6-Phenoxypyrid-2-yl)ethyl 3-(2-chloro-3,3,3-trifluoroprop-1-en-1-yl)-2,2-dimethylcyclopropanecarboxylate ;

1-[6-(4-chlorophenoxy)pyrid-2-yl]ethyl 3-(2-chloro-3,3,3-trifluoroprop-1-en-1-yl)-2,2-dimethylcyclopropanecarboxylate ;

1-(6-phenoxypyrid-2-yl)ethyl 3-(2,3,3,3-tetrafluoroprop-1-en-1-yl)-2,2-dimethylcyclopropanecarboxylate ; and

1-(6-phenoxypyrid-2-yl)ethyl 3-(2-bromo-3,3,3-trifluoroprop-1-en-1-yl)-2,2-dimethylcyclopropanecarboxylate ; and stereoisomers thereof.

5. A compound selected from the group of compounds consisting of :

(RS)-1-(6-phenoxypyrid-2-yl)ethyl (1RS,cis)-3-(Z-2-chloro-3,3,3-trifluoroprop-1-en-1-yl)-2,2-dimethylcyclopropanecarboxylate, and the more polar pair of enantiomers derived by high pressure liquid

chromatographic separation thereof ;
(RS)-1-(6-phenoxypyrid-2-yl)ethyl (1R,cis)-3-(Z-2-chloro-3,3,3-trifluoroprop-1-en-1-yl)-2,2-dimethylcyclopropanecarboxylate ;
(RS)-1-(6-phenoxypyrid-2-yl)ethyl (1R,trans)-3-(Z-2-chloro-3,3,3-trifluoroprop-1-en-1-yl)-2,2-dimethylcyclopropanecarboxylate ;
(RS)-1-(6-phenoxypyrid-2-yl)ethyl (1RS,trans)-3-(Z-2-chloro-3,3,3-trifluoroprop-1-en-1-yl)-2,2-dimethylcyclopropanecarboxylate ;
(RS)-1-[6-(4-chlorophenoxy)pyrid-2-yl]ethyl (1RS,cis)-3-(Z-2-chloro-3,3,3-trifluoroprop-1-en-1-yl)-2,2-dimethylcyclopropanecarboxylate ;
(RS)-1-(6-phenoxypyrid-2-yl)ethyl (1RS,cis)-3-(Z-2,3,3,3-tetrafluoroprop-1-en-1-yl)-2,2-dimethylcyclopropanecarboxylate ;
(RS)-1-(6-phenoxypyrid-2-yl)ethyl (1RS, trans)-3-(Z-2,3,3,3-tetrafluoroprop-1-en-1-yl)-2,2-dimethylcyclopropanecarboxylate ;
(RS)-1-(6-phenoxypyrid-2-yl)ethyl (1RS,cis)-3-(Z-2-bromo-3,3,3-trifluoroprop-1-en-1-yl)-2,2-dimethylcyclopropanecarboxylate ; and
(RS)-1-(6-phenoxypyrid-2-yl)ethyl (1RS,trans)-3-(Z-2-bromo-3,3,3-trifluoroprop-1-en-1-yl)-2,2-dimethylcyclopropanecarboxylate.

6. An insecticidal and acaricidal composition comprising an insecticidally and acaricidally effective amount of the compound of claim 1 in association with an insecticidally and acaricidally inert diluent or carrier.

7. A method of combating insect and acarine pests at a locus which comprises treating the locus with an insecticidally and acaricidally effective amount of the composition of claim 7.

8. The method of claim 7 wherein the locus is a growing crop.

9. A process for preparing a compound according to claim 1 wherein
(a) an acid of formula (II) :

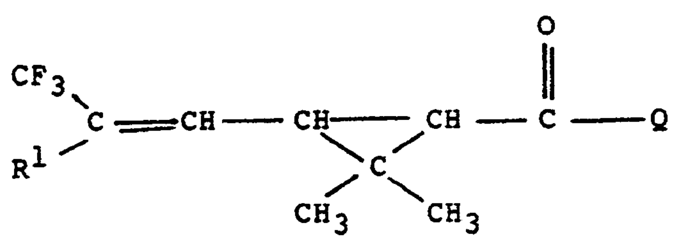

(II)

wherein $R^1$ has any of the meanings driven in claim 1 and Q represents the hydroxy group, is reacted with an alcohol of formula (III) :

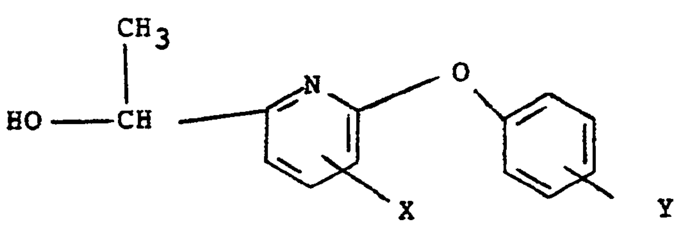

(III)

wherein X and Y have any of the meanings driven in claim 1, the reaction taking place in the presence of an acid catalyst or a dehydrating agent ; or
(b) an acid halide of formula (II) wherein Q represents a halogen atom and $R^1$ has any of the meanings given in claim 1, is reacted with an alcohol of formula (III) wherein X and Y have any of the meanings given in claim 1, the reaction taking place in the presence of a base ; or
(c) an acid of formula (II) wherein Q represents the hydroxy group and $R^1$ has any of the meanings given in claim 1, is reacted with a compound of formula (IV) :

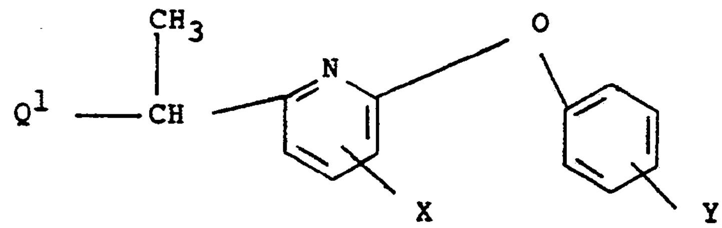

(IV)

wherein $Q^1$ represents a halogen atom and X and Y have any of the meanings driven in claim 1, or with the quaternary ammonium salts derived from the reaction between such halides and a tertiary amine ; or (d) a lower alkyl ester of formula (II) wherein Q represents a lower alkoxy group containing up to six carbon atoms and $R^1$ has any of the meanings given in claim 1, is heated with an alcohol of formula (III), wherein X and Y have any of the meanings given in claim 1, to effect a transesterification reaction.

**Patentansprüche**

1. Verbindung der Formel

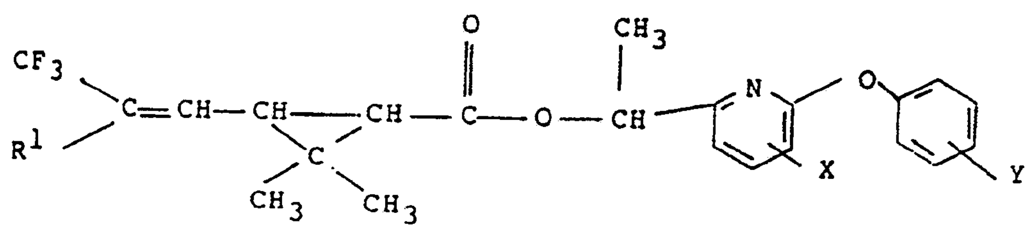

und Stereoisomere davon, worin $R^1$ ausgewählt ist aus Chlor, Brom und Fluor, X ausgewählt ist aus Wasserstoff und Halogen und Y ausgewählt ist aus Wasserstoff und Halogen.

2. Verbindung nach Anspruch 1, worin Y ausgewählt ist aus Wasserstoff und Chlor, und Stereoisomere davon.

3. Verbindung nach Anspruch 1 oder 2, worin $R^1$ für Chlor oder Brom steht, und Stereoisomere davon.

4. Verbindung, welche ausgwählt ist aus
3-(2-Chloro-3,3,3-trifluoroprop-1-en-yl)-2,2-dimethylcyclopropan-carbonsäure-1-(6-phenoxypyrid-2-yl)ethyl-ester,
3-(2-Chloro-3,3,3-trifluoroprop-1-en-yl)-2,2-dimethylcyclopropan-carbonsäure-1-[6-(4-chlorophenoxy)pyrid-2-yl]ethyl-ester,
3-(2,3,3,3-Tetrafluoroprop-1-en-1-yl)-2,2-dimethylcyclopropancarbonsäure-1-(6-phenoxypyrid-2-yl) ethyl-ester und
3-(2-Bromo-3,3,3-trifluoroprop-1-en-1-yl)-2,2-dimethylcyclopropan-carbonsäure-1-(6-phenoxypyrid-2-yl)ethyl-ester ; sowie Stereoisomeren davon.

5. Verbindung, welche ausgewählt ist aus
(1RS,cis)-3-(Z-2-Chloro-3,3,3-trifluoroprop-1-en-1-yl)-2,2,-dimethylcyclopropan-carbonsäure-(RS)-1-(6-phenoxypyrid-2-yl)-ethyl-ester und dem polareren Enantiomerenpaar, das durch Trennung mittels Hochdruckflüssigkeitschromatografie erhältlich ist ;
(1R, cis)-3-(Z-2-Chloro-3,3,3-trifluoroprop-1-en-1-yl)-2,2,-dimethylcyclopropan-carbonsäure-(RS)-1-(6-phenoxypyrid-2-yl)ethyl-ester,
(1R,trans)-3-(Z-2-Chloro-3,3,3-trifluoroprop-1-en-1-yl)-2,2-dimethylcyclopropan-carbonsäure-(RS)-1-(6-phenoxypyrid-2-yl)ethyl-ester,
(1RS, trans)-3-(Z-2-Chloro-3,3,3-trifluoroprop-1-en-1-yl)-2,2-dimethylcyclopropan-carbonsäure-(RS)-1-(6-phenoxypyrid-2-yl)-ethyl-ester,
(1RS,cis)-3-(Z-2-Chloro-3,3,3-trifluoroprop-1-en-1-yl)-2,2, dimethylcyclopropan-carbonsäure-(RS)-1-[6-(4-chlorophenoxy)-pyrid-2-yl]ethyl-ester,
(1RS, cis)-3-(Z-2,3,3,3-Tetrafluoroprop-1-en-1-yl)-2,2-dimethylcyclopropan-carbonsäure-(RS)-1-(6-phenoxypyrid-2-yl)ethyl-ester,
(1RS, trans)-3-(Z-2,3,3,3-Tetrafluoroprop-1-en-1-yl)-2,2-dimethylcyclopropan-carbonsäure-(RS)-1-(6-phenoxypyrid-2-yl)ethyl-ester,
(1RS, cis)-3-(Z-2-Bromo-3,3,3-trifluoroprop-1-en-1-yl)-2,2,dimethylcyclopropan-carbonsäure-(RS)-1-(6-phenoxypyrid-2-yl)-ethyl-ester und
(1RS, trans)-3-(Z-2-Bromo-3,3,3-trifluoroprop-1-en-1-yl)-2,2-dimethylcyclopropan-carbonsäure-(RS)-1-(6-phenoxypyrid-2-yl)ethyl-ester.

6. Insecticide und acaricide Zusammensetzung, welche eine insecticid und acaricid wirksame Menge einer Verbindung nach Anspruch 1 gemeinsam mit einem insecticid und acaricid inerten Verdünnungs- oder Trägermittel enthält.

7. Verfahren zur Bekämpfung von Insekten- und Milbenschädlingen an einem bestimmten Ort, bei welchem der Ort mit einer insecticid und acaricid wirksamen Menge einer Zusammensetzung nach Anspruch 7 behandelt wird.

8. Verfahren nach Anspruch 7, bei welchem der Ort aus wachsenden Feldfrüchten besteht.

9. Verfahren zur Herstellung einer Verbindung nach Anspruch 1, bei welchem
(a) eine Säure der Formel II,

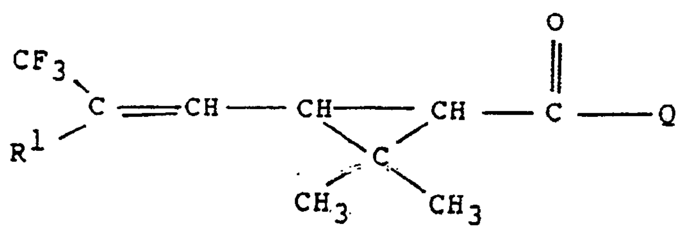

(II)

worin $R^1$ die in Anspruch 1 angegebene Bedeutung besitzt und Q für eine Hydroxygruppe steht, mit einem Alkohol der Formel III,

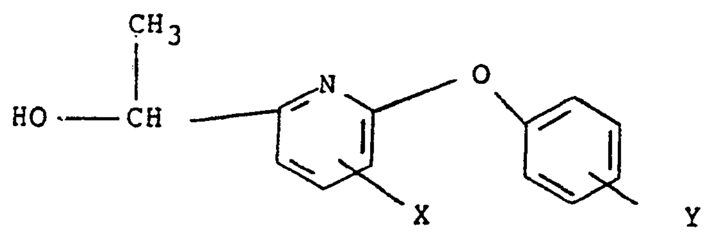

(III)

worin X und Y die in Anspruch 1 angegebenen Bedeutungen besitzen, in Gegenwart eines sauren Katalysators oder eines Dehydratisierungsmittels umgesetzt wird ; oder
(b) ein Säurehalogenid der Formel II, worin Q für ein Halogenatom steht und $R^1$ die in Anspruch 1 angegebene Bedeutung besitzt, mit einem Alkohol der Formel III, worin X und Y die in Anspruch 1 angegebenen Bedeutungen besitzen, in Gegenwart einer Base umgesetzt wird ; oder
(c) eine Säure der Formel II, worin Q für eine Hydroxygruppe steht und $R^1$ die in Anspruch 1 angegebene Bedeutung besitzt, mit einer Verbindung der Formel IV,

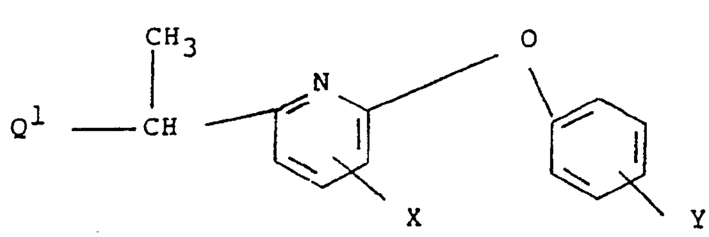

(IV)

worin $Q^1$ für ein Halogenatom steht und X und Y die in Anspruch 1 angegebenen Bedeutungen besitzen, oder mit dem quätemären Ammoniumsalz, das durch Reaktion zwischen einem solchem Halogenid und einem tertiären Amin erhältlich ist, umgesetzt wird ; oder
(d) ein Niederalkylester der Formel II, worin Q für eine Niederalkoxygruppe mit bis zu 6 Kohlenstoffatomen steht und $R^1$, die in Anspruch 1 angegebene Bedeutung besitzt, mit einem Alkohol der Formel III, worin X

und Y die in Anspruch 1 angegebenen Bedeutungen besitzen, zur Bewirkung einer Umesterung erhitzt wird.

## Revendications

1. Composé de formule :

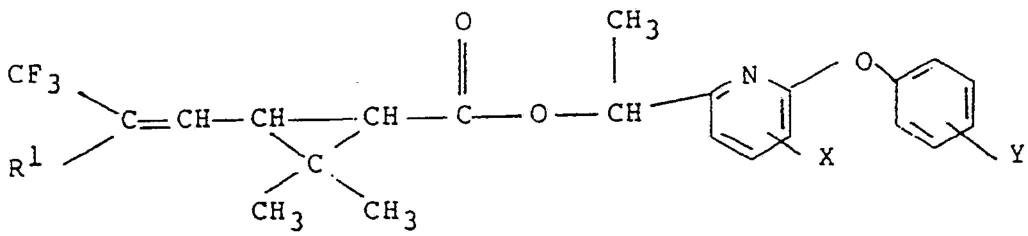

et ses stéréo-isomères, formule dans laquelle $R^1$ est choisi entre le chlore, le brome et le fluor, X est choisi entre l'hydrogène et un halogène et Y est choisi entre l'hydrogène et un halogène.

2. Composé suivant la revendication 1, dans lequel Y est choisi entre l'hydrogène et le chlore, et ses stéréo-isomères.

3. Composé suivant la revendication 1 ou la revendication 2, dans lequel $R^1$ représente le chlore ou le brome, et ses stéréo-isomères.

4. Composé choisi dans le groupe des composés comprenant :
le 3-(2-chloro-3,3,3-trifluoroprop-1-ène-1-yl)-2,2-diméthylcyclopropanecarboxylate de 1-(6-phénoxypyrid-2-yl)éthyle ;
le 3-(2-chloro-3,3,3-trifluoroprop-1-ène-1-yl)-2,2-diméthylcyclopropanecarboxylate de 1-[6-(4-chlorophénoxy)pyrid-2-yl]éthyle ;
le 3-(2,3,3,3-tétrafluoroprop-1-ène-1-yl)-2,2-diméthylcyclopropanecarboxylate de 1-(6-phénoxypyrid-2-yl)éthyle ; et
le 3-(2-bromo-3,3,3-trifluoroprop-1-ène-1-yl)-2,2-diméthylcyclopropanecarboxylate de 1-(6-phénoxypyrid-2-yl)éthyle ; et leurs stéréro-isomères.

5. Composé choisi dans le groupe des composés comprenant :
le (1RS,cis)-3-(Z-2-chloro-3,3,3-trifluoroprop-1-ène-1-yl)-2,2-diméthylcyclopropanecarboxylate de (RS)-1-(6-phénoxypyrid-2-yl)éthyle et la paire la plus polaire d'énantiomères obtenus par séparation par chromatographie en phase liquide sous haute pression ;
le (1R,cis)-3-(Z-2-chloro-3,3,3-trifluoroprop-1-ène-1-yl)-2,2-diméthylcyclopropanecarboxylate de (RS)-1-(6-phénoxypyrid-2-yl)éthyle ;
le (1R,trans)-3-(Z-2-chloro-3,3,3-trifluoroprop-1-ène-1-yl)-2,2-diméthylcyclopropanecarboxylate de (RS)-1-(6-phénoxypyrid-2-yl)éthyle ;
le (1RS,trans)-3-(Z-2-chloro-3,3,3-trifluoroprop-1-ène-1-yl)-2,2-diméthylcyclopropanecarboxylate de (RS)-1-(6-phénoxypyrid-2-yl)éthyle ;
le (1RS,cis)-3-(Z-2-chloro-3,3,3-trifluoroprop-1-ène-1-yl)-2,2-diméthylcyclopropanecarboxylate de (RS)-1-[6-(4-chlorophénoxypyrid-2-yl)éthyle ;
le (1RS,cis)-3-(Z-2,3,3,3-tétrafluoroprop-1-ène-1-yl)-2,2-diméthylcyclopropanecarboxylate de (RS)-1-(6-phénoxypyrid-2-yl)éthyle ;
le (1RS,trans)-3-(Z-2,3,3,3-tétrafluoroprop-1-ène-1-yl)-2,2-diméthylcyclopropanecarboxylate de (RS)-1-(6-phénoxypyrid-2-yl)éthyle ;
le (1RS,cis)-3-(Z-2-bromo-3,3,3-trifluoroprop-1-ène-1-yl)-2,2-diméthylcyclopropanecarboxylate de (RS)-1-(6-phénoxypyrid-2-yl)éthyle ; et
le (1RS,trans)-3-(Z-2-bromo-3,3,3-trifluoroprop-1-ène-1-yl)-2,2-diméthylcyclopropanecarboxylate de (RS)-1-(6-phénoxypyrid-2-yl)éthyle.

6. Composition insecticide et acaricide, comprenant une quantité à effet insecticide et acaricide du composé suivant la revendication 1 en association avec un diluant ou support inerte du point de vue insecticide ou acaricide.

7. Procédé pour combattre des insectes et des acariens parasites en un lieu, qui consiste à traiter le lieu avec une quantité à effet insecticide et acaricide de la composition de la revendication 7.

8. Procédé suivant la revendication 7, dans lequel le lieu est une culture en cours de croissance.

9. Procédé de préparation d'un composé suivant la revendication 1, dans lequel
(a) un acide de formule (II) :

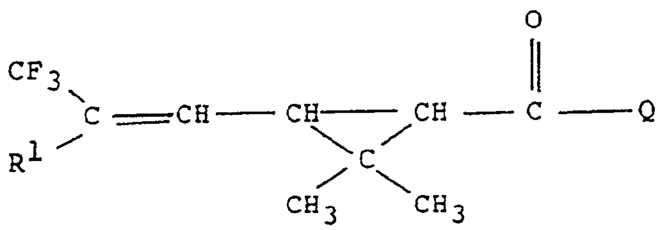

(II)

dans laquelle $R^1$ a l'une quelconque des définitions données dans la revendication 1 et Q représente le groupe hydroxy, est amené à réagir avec un alcool de formule (III) :

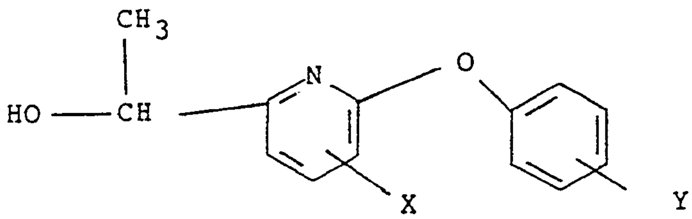

(III)

dans laquelle X et Y ont l'une quelconque des définitions indiquées dans la revendication 1, la réaction ayant lieu en présence d'un catalyseur acide ou d'un agent déshydratant ; ou
(b) un halogénure d'acide de formule (II) dans laquelle Q représente un atome d'halogène et $R^1$ a l'une quelconque des définitions données dans la revendication 1, est amené à réagir avec un alcool de formule (III) dans laquelle X et Y ont l'une quelconque des définitions indiquées dans la revendication 1, la réaction ayant lieu en présence d'une base ; ou
(c) un acide de formule (II) dans laquelle Q représente le groupe hydroxy et $R^1$ a l'une quelconque des définitions indiquées dans la revendication 1 est amené à réagir avec un composé de formule (IV) :

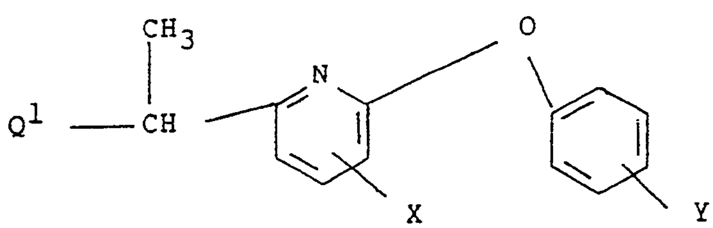

(IV)

dans laquelle $Q^1$ représente un atome d'halogène et X et Y ont l'une quelconque des définitions indiquées dans la revendication 1, ou avec les sels d'ammonium quaternaire obtenus par réaction entre ces halogénures et une amine tertiaire ; ou bien
(d) un ester alkylique inférieur de formule (II) dans laquelle Q représente un groupe alkoxy inférieur contenant jusqu'à 6 atomes de carbone et $R^1$ a l'une quelconque des définitions indiquées dans la revendication 1 est chauffé avec un alcool de formule (III) dans laquelle X et Y ont l'une quelconque des définitions indiquées dans la revendication 1 pour effectuer une réaction de transestérification.